# EUROPEAN PATENT SPECIFICATION

(11) **EP 0 444 856 B1**
(45) Date of publication and mention of the grant of the patent: **03.09.1997**
(21) Application number: 91301506.1
(22) Date of filing: 26.02.1991
(51) Int. Cl.: G01N 33/68, C12P 21/08

(54) **Diagnostic assay for Alzheimer's disease**
Verfahren zum Nachweis der Alzheimer-Krankheit
Procédé pour la détection de la maladie d'Alzheimer

(30) Priority: 26.02.1990 US 485149
(43) Date of publication of application: 04.09.1991
(73) Proprietor: ALBERT EINSTEIN COLLEGE OF MEDICINE OF YESHIVA UNIVERSITY, Bronx New York 10461 (US)
(72) Inventor: Ghanbari, Hossein A., Libertyville, Illinois 60048 (US); Davies, Peter, Rye, New York 10580 (US)
(74) Representative: Vossius, Volker, Dr.

(56) References cited:
- EP-A- 0 411 974
- WO-A-89/03993
- WO-A-89/06242
- US-A- 4 666 829
- ANNALS OF NEUROLOGY; vol. 22, no. 4, 1987, Boston, US, pp. 521-526; B. WOLOZIN et al.:"Alzheimer-related neuronal protein A68: specificity and distribution"
- SCIENCE; vol. 232, no. 4750, 2 May 1986, Lancaster, PA, US; pp. 648-650; B.L. WOLOZIN et al.: "A neuronal antigen in the brains of Alzheimer patients"
- ANALYTICAL CHEMISTRY; vol. 59, no. 20, 15 October 1987, Columbus, Ohio, US, pp. 1203A-1204A; M. WARNER: "Diagnosis of Alzheimer's disease"
- JOURNAL OF CLINICAL LABORATORY ANALYSIS; vol. 4, 1990, New York, US, pp. 189-192; H.A. GHANBARI et al.: "A sandwich enzyme immunoassay for detecting and measuring Alzheimer's disease-associated proteins in human brain tissue"

## Description

This invention relates to Alzheimer's disease and methods for diagnosing Alzheimer's disease using antigens and antibodies related to Alzheimer's disease.

Alzheimer's disease is a progressive neurodegenerative disorder affecting 7% of the population over 65 years of age and characterized clinically by progressive loss of intellectual function and pathologically by a continuing loss of neurons from the cerebral cortex. More specifically, this pathological impairment usually is correlated with numbers of neuritic plaques in the neocortex and with the loss of presynaptic markers of cholinergic neurons. Neuritic plaques are composed of degenerating axons and nerve terminals, as well as possible astrocytic elements, and these plaques often exhibit a central core.

Another characteristic pathological feature of Alzheimer's disease is development of neurofibrillary tangles. A neurofibrillary tangle is an intraneuronal mass composed of normal intermediate filaments and paired helical filaments having unusual properties, which twist and form tangles. Neurofibrillary tangles presumably are comprised of several different proteins.

Neurochemical studies show yet another pathological feature of the disease; many neurotransmitter systems are affected by Alzheimer's. The most consistent and severely affected system is that of the cholinergic neurons located in the Nucleus Basalis of Meynert. In addition, somatostatin, substance P and corticotropin releasing factor are reduced in amount by the disease process.

None of the above-mentioned pathologic structures, neurochemical alterations, neuritic plaques and neurofibrillary tangles are unique to Alzheimer's disease. These impairments also occur in the brains of normal aged individuals and are associated with other diseases such as Guam Parkinson Disease, Dementia Pugilistica and Progressive Supra-nuclear Palsy. For example, paired helical filaments, the twisted filaments that form the tangles and fill the neurities of plaques, also occur in certain tangles associated with other diseases such as Pick's Disease. In fact, immunologic studies have shown that epitopes of paired helical filaments exist in Pick bodies, the spherical structures found in affected neurons in the temporal cortex of brains affected by Pick's Disease. In addition to the non-specifity of these impairments to Alzheimer's disease, the densities of neurofibrillary tangles and neuritic plaques within the cerebral cortex of an Alzheimer's patient do not necessarily correlate with the stages of the illness.

Accordingly, the diagnosis of Alzheimer's disease is very difficult. Ante-mortem diagnosis of the disease is primarily by exclusion. A recent article entitled, "The Neurochemistry of Alzheimer's disease and Senile Dementia", by Peter Davies in Medicinal Research Reviews, Vol. 3, No. 3, pp. 221-236 (1983), discusses Alzheimer's disease and at page 223 states:

The problem in the diagnosis of Alzheimer's disease is that there is no positive test: the clinician has to rule out other causes of dementia such as strokes, microvascular disease, brain tumors, thyroid dysfunction, drug reactions, severe depression and a host of other conditions that can cause intellectual deficits in elderly people. Only when all of these problems have been eliminated as a cause of the symptoms should a diagnosis of Alzheimer's disease be accepted.

Post-mortem diagnosis is based on determination of the number of neuritic plaques and tangles in brain tissue which has been stained to visualize these plaques and tangles. However, such diagnostic methods, based on neurohistopathological studies, require extensive staining and microscopic examination of several brain sections. Moreover, since the plaques and tangles also may occur in the brains of normal, elderly individuals or individuals with other diseases, a more definitive and reliable method for making the diagnosis on brain tissue is desirable.

Monoclonal antibodies to Alzheimer's disease, may be used in diagnostic tests for Alzheimer's disease. These antibodies, singularly referred to herein as "Alzheimer antibody", react against brain tissue homogenates from patients with Alzheimer's disease and bind to a highly selective protein marker for this disorder, described herein as the "Alzheimer antigen". Alzheimer antibody consistently stains neurons in Alzheimer's disease brains and such immunocytochemical staining has established that the Alzheimer antibody recognizes an early cytological change, specific for Alzheimer S disease, which precedes the formation of neurofibrillary tangles and neuritic plaques.

EP 0 411 974 discloses a monoclonal antibody specific to Alzheimer's disease and a method of detection using said antibody. In Annals of Neurology (1987) 22(4) 521-526 the use of monoclonal antibody ALZ50 to recognise antigen A68 is taught, the complex being detected by a peroxidase-coupled or ¹²⁵I coupled goat antimouse antibody.
In Science (1986) 233 648-650 the use of monoclonal antibody ALZ 50 is also demonstrated for recognising antigen A68, the complex being detected by a peroxidase-coupled goat antimouse antibody.

In Anal.Chemistry (1987) 59(20) 1203A-1204A it is stated that an immunoassay, competitive or sandwich, has been developed using antigen developed brain protein tangles of Alzheimer's disease sufferers.

WO 89/03993, US 4 666 829 and WO 89/06242 all disclose that immunoassays for antigens specific to Alzheimer's disease are known.

The present invention provides a specific, sensitive and simple sandwich immunoassay for diagnosis of Alzheimer's disease and overcomes the drawbacks of the prior art which require a diagnosis based on a process of elimination of other disorders.

Accordingly, it is an object of the present invention to provide a method for determining the presence of antigens specific to Alzheimer's disease in a sample comprising the steps of:
a. contacting the sample with a first antibody directed to an antigenic determinant on the antigen and capable of binding to the antigen so as to produce a first complex;
b. recovering the first complex from the sample;
c. contacting the first complex so recovered with at least one second antibody directed to a second antigenic determinant on the antigen, wherein said second antigenic determinant may be the same as said first antigenic determinant, and capable of binding to the antigen present in the first complex so as to bind the antigen and produce a second complex consisting of the antigen bound to the first and second antibody; and
d. detecting the presence of the second complex to thereby determine the presence of antigen specific to Alzheimer's disease in the sample.

Another object of the present invention is to provide a method for diagnosing Alzheimer's disease using pre-mortem or post-mortem brain tissue or cerebral spinal fluid from a patient.

An additional object of the present invention is to provide a method for detecting and measuring antigen specific to Alzheimer's disease in a sample comprising the steps of:
a. contacting the sample with a first antibody directed to an antigenic determinant on the antigen and capable of binding to the antigen so as to produce a complex;
b. recovering the resulting complex from the sample;
c. contacting the complex so recovered with a detectable second antibody directed to a second antigenic determinant on the antigen, wherein said second antigenic determinant may be the same as said first antigenic determinant, and capable of binding to the antigen present in the complex so as to bind the antigen and
d. detecting and measuring the second antibody bound to the antigen to thereby detect and measure antigen specific to Alzheimer's disease.

As indicated above, the term "Alzheimer antigen" is used herein to refer to antigens specific to Alzheimer patients. Alzheimer antigen has been described in detail in U.S. Patent Application Serial No. 100,980, incorporated herein by reference. The term Alzheimer's Disease Associated Proteins ("ADAP") is used interchangeably herein with Alzheimer antigen and is preferable when the protein aspect of the Alzheimer antigen is being discussed. The term "Alzheimer antibody" is used herein to identify an antibody which recognizes Alzheimer antigen.

In one aspect, the present invention concerns a method for determining the presence in a sample of antigen specific to Alzheimer's disease. The method involves contacting a sample with a combination of at least two different antibodies, one of which is detectable and each of which is directed to an antigenic determinant on the antigen and is capable of binding to the antigen. The presence of the detectable antibody or complex is determined and thereby the presence in the sample of the antigen specific to Alzheimer's disease is determined.

In another aspect, the present invention is a method for detecting and measuring the amount of antigen specific to Alzheimer's disease in a sample.

A further aspect of the present invention is a method for diagnosis of Alzheimer's disease based upon the sandwich immunoassay of the present invention.

Figure 1 illustrates the sandwich immunoassay configuration of the present invention for the diagnosis of Alzheimer's disease.

Figure 2 shows the relationship between absorbance and micrograms IgG/ bead.

Figure 3 shows titration curves for Alzheimer's disease brain homogenates and normal brain homogenates.

Figure 4A is a full scale graph scattergram plot of the ADAP concentrations for each diagnostic category; NC: Normal Control; NDC: Neurological Disease Control; AD: Alzheimer's Disease; SDAT: Senile Dementia of Alzheimer's Type; D/AD: Down's Syndrome with AD Neurohistology; Non-AD group includes NC and NDC, and AD group includes AD, SDAT and D/AD. Closed circles are values for 65 and older cases, open circles are values for under 65 cases. The circles on the 2.0 line are either 2.0 or higher (up to 16).

Figure 4B is a y-axis 10 -fold expansion of Figure 4A.

A method is provided for determining the presence in a sample of antigen specific to Alzheimer's disease. The method comprises contacting the sample with a first antibody directed to an antigenic determinant on the antigen and capable of binding to the antigen so as to produce a first complex. The resulting first complex then is recovered from the sample and contacted with at least one second antibody directed to a second antigenic determinant on the antigen, which second antigenic determinant may be the same as the first antigenic determinant due to the multiepitopic nature of the antigenic entity, and capable of binding to the antigen present in the first complex so as to bind the antigen and produce a second complex consisting of antigen bound to the first and second antibodies. The presence of the second complex is detected and, thereby, the presence of the antigen specific to Alzheimer's disease in the sample is determined.

Also provided is another method for determining the presence in a sample of antigen specific to Alzheimer's disease. The method comprises contacting the sample with a first antibody directed to an antigenic determinant on the antigen and capable of binding to the antigen so as to produce a complex. The resulting complex then is recovered from the sample and contacted with at least one detectable second antibody directed to a second antigenic determinant on the antigen, which second antigenic determinant may be the same as the first antigenic determinant due to the multiepitopic nature of the antigenic entity, and capable binding to the antigen present in the complex so as to bind the antigen. The presence of the second antibody so bound is detected and, thereby, the presence of the antigen specific to Alzheimer's disease in the sample is determined.

The sample tested may be brain tissue, pre or post-mortem, cerebral spinal fluid or blood. In a preferred embodiment, the sample comprises frontal or temporal brain cortical tissue since such tissue consistently has higher ADAP levels.

In a further preferred embodiment, the first antibody is a rabbit polyclonal antibody and the second antibody is at least one monoclonal antibody. The presently preferred monoclonal antibody is ALZ-50 secreted by a hybridoma on deposit at the American Type Culture Collection and catalogued as ATCC No. HB9205. Moreover, it is presently preferred that the first antibody be attached to a solid matrix, e.g., polystyrene beads.

A sample is obtained and contacted with a suitable amount of first antibody to produce a complex. The contact typically involves adding the sample to a column of polystyrene beads coated with the first antibody.

The complex which results from contacting the sample with the first antibody is separated from the sample by elution. However, other methods of recovery may be employed.

The recovered complex is contacted with at least one second antibody directed to an antigenic determinant on the antigen and capable of binding to the antigen in the complex. The antigenic determinant to which the second antibody is directed may be the same one as that to which the first antibody is directed due to the multiepitopic nature (i.e. repeating epitopes) of the antigenic entity. The conditions for effecting such contact are described herein and known to those skilled in the art.

The first or second antibody of the methods of the present invention may be made detectable by attaching an identifiable label to it. In a preferred embodiment, the second antibody is made detectable. The antibody preferably is made detectable by attaching to it an enzyme conjugated to an appropriate substrate which, in turn, catalyzes a detectable reaction. The enzyme may be horseradish peroxidase, beta-galactosidase or alkaline phosphotase. Other means of detection of the antibody include attaching a fluorescent or radiolabel thereto. Alternatively, the antibody may be detected by use of another antibody directed to it, the other antibody being labeled or having an enzyme substrate bound to it.

The presence of the detectable antibody bound to the antigen of the complex consisting of antigen bound to the first and second antibody may be readily detected using well-known techniques. Thus, if the detectable antibody is linked to an enzyme conjugated to an appropriate substrate, the optical density of the detectable bound antibody is determined using a quantum spectrotometer. If the detectable antibody is fluorescently labeled, the fluorescent emission may be measured or detected using a fluorometer technique. In a similar manner, if the detectable antibody is radioactively labeled, the bound antibody may be detected using a radioactivity detection techniques. By comparing the results obtained using the above-described methods on the test sample with those obtained using the methods on a control sample, the presence of the antigen specific to Alzheimer disease may be determined.

The above described methods for determining the presence of Alzheimer antigen may be made quantitative as well. One such method for quantitatively determining, i.e. for detecting and measuring, Alzheimer antigen in a sample comprises contacting the sample with a first antibody directed to an antigenic determinant on the antigen and capable of binding to the antigen so as to produce a complex. The resulting complex then is recovered from the sample and contacted with at least one detectable second antibody directed to a second antigenic determinant on the antigen, wherein said second antigenic determinant may be the same as said first antigenic determinant, and capable binding to the antigen present in the complex so as to bind the antigen. The second antibody bound to the antigen is detected and measured and in so doing, the antigen specific to Alzheimer's disease is thereby detected and measured.

In this method for detecting and measuring Alzheimer antigen, the first antibody may be a monoclonal antibody and the second detectable antibody a polyclonal antibody. Alternatively, all antibodies employed may be monoclonal antibodies. In still another embodiment, the first antibody and not the second may be made detectable.

The methods for qualitatively or quantitatively determining the Alzheimer antigen may be used in the diagnosis of Alzheimer's disease. Utilization of the methods of the present invention is advantageous over prior art methods because the present invention provides simple, sensitive, very specific methods for detecting Alzheimer's antigen. The configuration of this assay minimizes complications caused by the cross-reactants from both Alzheimer's disease and normal brain homogenates. In particular, while the first and second antibody each crossreact with other proteins, they do not crossreact with the same proteins and, therefore, the specificity of the assay is greatly enhanced. In addition, there is strong evidence that ADAP is better suited for a sandwich immunoassay because ADAP is present in aggregate form and, hence, multi-epitopic, in contrast to the crossreactive proteins which are soluble and appear to have only one epitope. Moreover, the assay is linear up to 0.5 absorbance unit (r=0.9), reproducible (CV less than 10%), sensitive, and specific. With preformulated reagents and standard supplies, the assay is simple and rapid; 120 data points readily can be generated in about 4 hours.

### Experimental Details

### Materials:

ALZ-50, Rabbit anti-ALZ-50, casein, Quantum, QuikWash System, Reaction Plates, Reaction Tubes, OPD Reagent, Gentamicin Sulfate, Nipasept, H₂SO₄ Reagent, 2.54 x 10⁻²m (1/4") beads and TDx microcentrifuge were obtained from Abbott Laboratories; HRPO Goat anti IgM conjugate from Fisher Scientific; microfuge tubes with matching pestle, and pestle driver from Kontes; Protein A from BioRad; BSA, Tween (a registered Trade Mark) 20 and EGTA from Sigman.

### Tissue Preparation:

Frozen brain specimens were thawed at room temperature until they had a firm consistency to prevent splintering during cutting. About 50 to 150 mg of cortical tissue was placed into a microfuge tube. (Frontal and temporal brain cortical tissue consistently has higher ADAP levels.) Cold homogenization buffer (0.1mM Tris, 150 mM NaCl containing EGTA Gentamicin sulfate and Nipaset as preservatives) then was added at 4 uL per mg of tissue. The tissue was homogenized in the microfuge tube for about one minute using the matching pestle driven by hand or mechanical motor. The particulate matter was removed from the homogenate by centrifuging it for one minute in a TDx centrifuge or the equivalent. The tissue was processed at 4°C throughout the preparation. (-70°C is recommended for longer storage of the homogenate).

### Antibody Preparation:

### ALZ-50:

The production of monoclonal antibodies is well-known in the art and has been described in many articles including "High Frequencies of Antigen-Specific Hybridomas; Dependent on Immunization Perimeters and Prediction by Spleen Cell Analysis", by Christian Stahli et al., Journal of Immunization Methods, Vol. 32, pp. 297-304 (1980) and "Production of Monoclonal Antibodies; Strategy and Techniques" by S. Fazekas de St. Groth and Dolores Scheidegger, Journal of Immunization Methods, Vol. 35, pp. 1-21 (1983). Furthermore, descriptions of procedures and critical steps are readily available from companies such as Microbiological Associates engaged in the business of supplying related products.

ALZ-50 is secreted by a hybridoma on deposit with the America Type Culture, Rockville, Maryland and cataloged as ATCC #HB9205.

### Rabbit Anti-ALZ IgG:

Alzheimer's disease brain homogenates were enriched for ADAP by multiple differential centrifugation steps and detergent extractions. Rabbits (Nos. 5359, 5432, 5433, 5434 and 5435) were immunized with highly enriched ADAP fraction and boosted according to a monthly schedule. Bleeds were screened by using ELISA in which the "immunogen" preparation was dried in the wells by forced air at 37°C. After desired titers were achieved, rabbit sera were purified by protein-A affinity chromatography. The Protein A purified IgG from the serum bleeds of these rabbits was used to coat the beads used in the assay.

More specifically, the following reagents were used in the preparation of Alzheimer disease brain homogenates enriched for ADAP:
[10x] Homogenate Buffer Stock
100 mM Tris
1.5 M NaCl
10 mM EGTA
pH 6.8

TBS - Tris Buffered Saline
0.01M Tris
0.9% NaCl
pH 7.5

100 mM PMSF/ ethanol (Protease inhibitor)
1% of total volume of brain homogenate mixture

The brain regions to be processed were identified and isolated. The tissue was stored at -80°C and placed at -20°C overnight. When ready, the tissue was slightly thawed at room temperature in biohazard hood to aid in dissection. However, the tissue still was kept on ice at all times. The dissected tissue sample then was accurately weighed and placed in a polyethylene container surrounded by ice. Four (4) volumes (ml/g) of cold homogenate buffer [1x] were added to tissue sample. PMSF was added to a total volume at a 1:100 dilution, 1mM final concentration. Prior to use, the Polytron homogenizer was cleansed with several alternating washes of distilled water, followed by ethanol and a final water rinse. The tissue and buffer were homogenized using a Polytron with 15-20 second blasts with equal intervals, at a setting of 5-6 on the instrument dial. (Adequate homogenization of larger samples may require longer blasts or a higher dial setting.) The sample vessel was surrounded by ice and kept as cool as possible during the homogenization process.

After complete homogenization of the tissue, 1 ml of the sample was aliquoted and saved for assay. The remainder was centrifuged at 20K x g for 20 to 25 minutes at 4°C. The resultant low speed supernatant was aspirated and the volume measured. It then was transferred to high speed centrifuge tubes. 1 ml of low speed supernatant was aliquoted and saved for assay (the low speed pellet contains considerable antibody reactivity and may be reextracted by resuspending to the original volume with cold TBS, and centrifuging as above). The high speed centrifuge tube was spun with volumes recorded, at 100K x g, 1 hour at 4°C. The high speed supernatant then was aspirated and 1 ml was aliquoted and saved for assay. The high speed pellet was carefully resuspended in cold TBS at a chosen concentration. Typically, (30x) stock solution was used for Alzheimer brain cortex tissue extract. Small volumes of this concentrated high speed pellet were aliquoted and stored at -80°C.

Samples from each step of the brain preparation were assayed for antibody reactivity and protein concentration.

### Bead Coating:

Protein-A purified IgG from the serum bleeds of the rabbits was used to coat the beads. The Abbott's standard 2.54 x 10⁻²m (1/4") polystyrene beads were coated by incubating them with rabbit anti-ALZ IgG in Tris Buffered Saline (pH 8.0) for 2 hours at room temperature and blocked with 4% BSA in phosphate buffered saline. Beads then were washed, overcoated with gelatine/ sucrose, dried, and stored at room temperature.

### The Sandwich Immunoassay:

The assay of the present invention detects and measures ADAP only. In general, the antigen (ADAP) effectively is captured by the polyclonal IgG coated on the beads. ALZ-50, used as the detection antibody, then is allowed to specifically bind to the immobilized antigen and the bound ALZ-50 subsequently is quantified by using an enzyme conjugated anti-mouse IgM (e.g., a horseradish peroxidase linked goat anti IgM and an appropriate substrate).

### Experiment 1

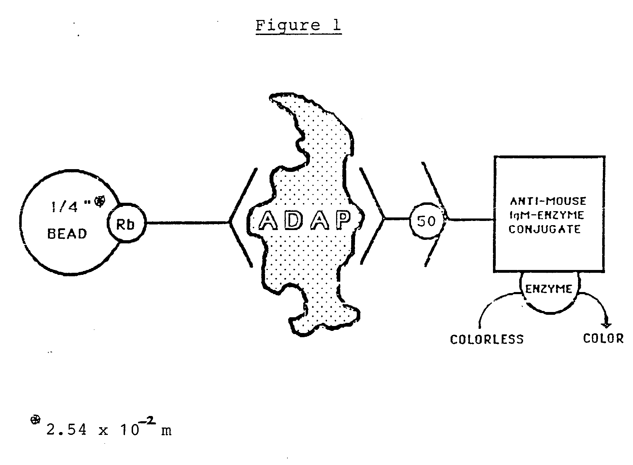

Figure 1 depicts the configuration of the assay. Post-mortem samples (tissue brain homogenates free of particulate matters) were diluted with sample buffer (1% BSA in PBS, pH 7.5, containing Gentamicin and Nipasept), usually 50 uL of the sample homogenate and 150 uL of the sample buffer. Each sample was run in duplicate. A known negative control, and a reagent blank (homogenization buffer instead of sample) were included on each plate. After adding one bead to each well, the plate was covered and incubated for 30 minutes at 37°C by floating it in a water bath. The beads were washed with distilled water twice using the QwikWash System, and then incubated with 200 uL of an ALZ-50 solution containing about 0.35 ug/ml of IgM (in PBS pH 7.5 containing 0.1% casein, 0.5% Tween (a registered Trade Mark) 20, Gentamicin and Nipasept) for 30 minutes at 37°C. The beads were washed again (twice), incubated with 200 uL of the HRPO conjugated goat anti mouse IgM diluted in the ALZ-50 diluent buffer containing 1% goat serum and incubated again for 30 minutes at 37°C. The beads were washed twice and transferred to the reaction test tubes according to instructions on the packaging. To each tube containing bead, 300 uL of OPD solution (prepare as per instructions) were added and incubated at room temperature for 30 minutes. The reaction was stopped by adding 1 mL of 1 N sulfuric acid to each tube and the solution was mixed by vortexing. The absorbance of each tube was determined using a Quantum spectrophotometer set to mode O and blanked with distilled water.

This assay procedure was used for detecting the ADAP in samples. However, if measurement of ADAP is desired for comparison of activity among several samples, the samples producing a background corrected absorbance of 0.5 or higher may be diluted serially and the dilution providing the highest absorbance under 0.5 may be used, making sure that the appropriate corrections are made for the dilutions. Moreover, although this assay normalizes the absorbance values to wet tissue weight, the user has the alternative of expressing the data per unit protein for ADAP measurement.

### Results and Discussion:

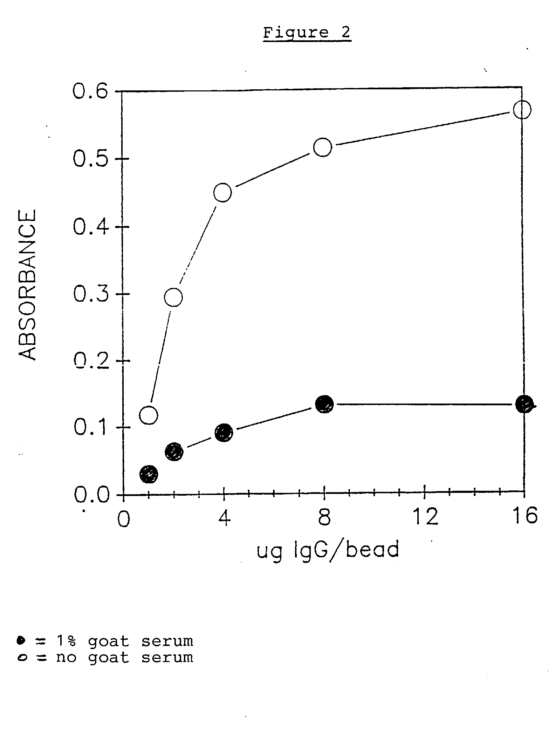

With respect to assay optimization, since room temperature was routinely used in manufacturing, only anti-ALZ rabbit IgG amount per bead, time, and pH were optimized. As can be seen in Figure 2, the background rapidly increased at levels over 2 ug/bead. However, at 4 ug/bead the background was acceptable and the signal approached maximum. Adding 1% goat serum to the conjugate solution considerably lowered the background. No further increase was observed beyond 2 hours and optimum pH was found to be 8.0. The blocking step involved a 30 min. incubation of antibody-coated bead in 4% BSA in PBS. Detection antibody and conjugate concentration were optimized by a standard checker board type titration. Incubation temperatures and durations were chosen to obtain acceptable signal reproducibility in the shortest possible time frame.

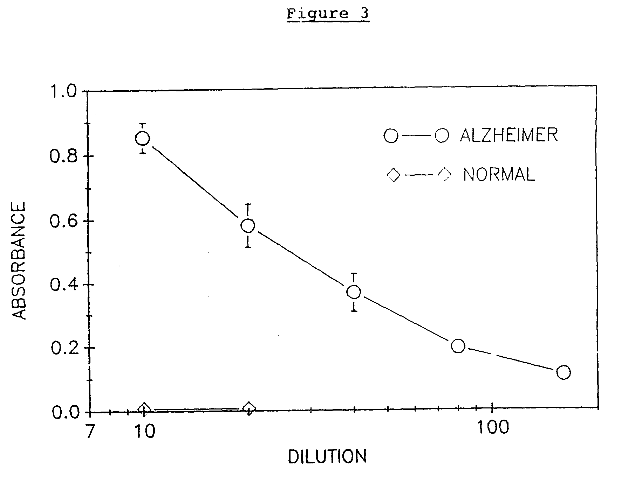

As to specificity and linearity, Figure 3 shows that there is no significant reactivity in the normal brain homogenate whereas ALZ-EIA activity in Alzheimer's disease dementia ("AD") brain homogenate titers down. The linear range of the curve is from 0.05 to 0.50.

Table 1 summarizes the assay's precision. It should be noted that signal for normals and background are essentially identical.

**Table 1**

| PRECISION STUDY | | | | |
|---|---|---|---|---|
| SAMPLE | N | MEAN | SD | MEAN OF WITHIN PLATE CV's |
| AD 1 | 30 | 0.384 | 0.040 | 9.3% |
| AD 2 | 40 | 0.458 | 0.064 | 9.8% |
| NORMAL 1 | 40 | 0.064 | 0.005 | 4.2% |
| NORMAL 2 | 40 | 0.058 | 0.004 | 4.2% |
| BACKGROUND | 40 | 0.056 | 0.007 | 8.5% |

AD 1 is a pool of AD brain homogenates. AD 2 is a homogenate from one AD brain specimen. Similarly, Normal 1 is a pool of non-AD brain homogenates and Normal 2 is a homogenate from one non-AD brain. Background represents assay runs in which homogenization buffer was used instead of brain homogenate. The mean is expressed as absorbance unit. The data was accumulated by 2 analysts over a period of 2 days. A total of 5 replicates were run per plate and mean of within plate CV's are reported in the Table.

### Experiment 2

The concentration of an Alzheimer's disease associated protein (ADAP) was measured in post-mortem brain tissue samples of temporal or frontal cortex from 111 human brains. Each of the patients from which the tissue was taken was evaluated clinically (e.g. diagnosis, age, gender and post mortem delay) and all the specimens used were evaluated pathologically prior to the biochemical analyses. There were 27 normal controls (NC), 28 neurological disease controls (NDC), and 53 Alzheimer's Disease (AD) of which seven were designated as Senile Dementia of Alzheimer's Type (SDAT), and three older Down's syndrome with Alzheimer's neuropathology D/AD. The cases in the neurological disease control category included: Parkinson's Disease (n=16), Multi-infarct Dementia (n=5), Huntington's Disease (n=2), Amyotrophic Lateral Sclerosis (n=2), Wernicke's Encephalopathy (n=1), and Korsakoff's syndrome (n=2). The AD group included the following categories: AD, SDAT and D/AD (Down's with AD neuropathology) and the non-AD group included the following categories: NC (normal control) and NDC.

The tissue sample, which ranged in wet weight from 20 to 200 mg, was mixed with 4 volumes of homogenization buffer (0.05 M TRIS HCl at pH=6.8, 1 mM EGTA and 150 mM NaCl) and then homogenized gently with a Kontes motorized pestle inside a standard 1.5 ml conical capped tube. The homogenate was centrifuged at 9500 x g for 5 minutes. A duplicate of 50 uL aliquot of the supernatant (equivalent to 10 mg wet tissue) was used in the assay. In each case, ADAP was measured and results were expressed as absorbance per mg of protein or as absorbance per 10 mg wet tissue weight. All absorbance readings for samples were corrected to net absorbance by subtracting absorbance reading of the assay when homogenization buffer was used instead of brain homogenate. Any negative net absorbance was reported as zero. The upper limit of the Quantum Spectrophotometer used was 2.0 absorbance. An absorbance over this limit was reported as 2.0; serial dilution indicated absorbance equivalent of up to 16. The data was analysed using a Student-t test and Spearman rank correlation coefficient.

### Results and Discussion:

The clinical data, patient information, pathological reports, and ADAP assay results for the 111 cases studied are summarized below in Table 2. Generally, the AD cases were older than NC cases (75.8 vs 60.7 years), but NDC cases were comparable to AD group (69.8 vs. 75.8). Considering only cases that were 65 or older, the average ages becomes comparable, 74.4,, 76.7, and 78.8 years for NC, NDC, and AD categories respectively. D/AD cases were all older than 50 (average age, 53). Post mortem time varied from as short as 1 hour to as long as 96; some cases did not have this information available. Pathological reports indicated that all specimens in the AD group (AD, SDAT, and D/AD categories) contained NP's ranging from mild to severe, but mostly severe. With respect to the severity of NP's in the non-AD group, NC cases had fewest NP's in the fewest cases, while NDC specimens had a reported density between NC and AD. All the cases in AD group were clinically demented, whereas none of the cases in the NC category were observed to have dementia. The majority of NDC cases were classified as demented.

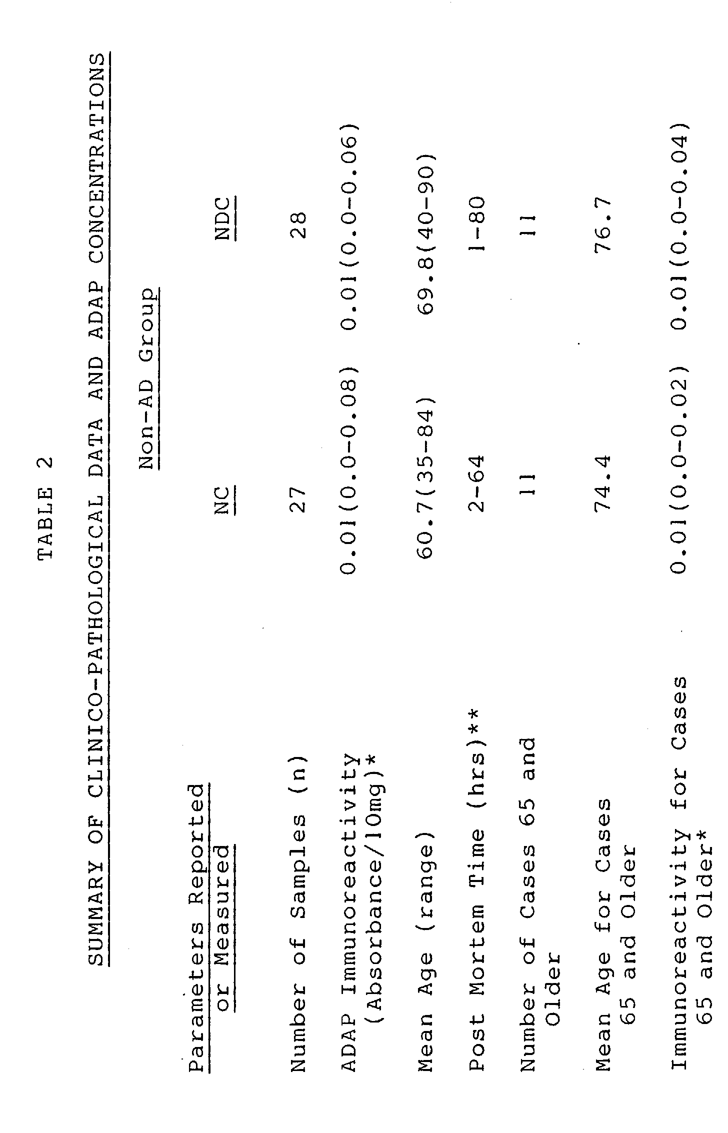

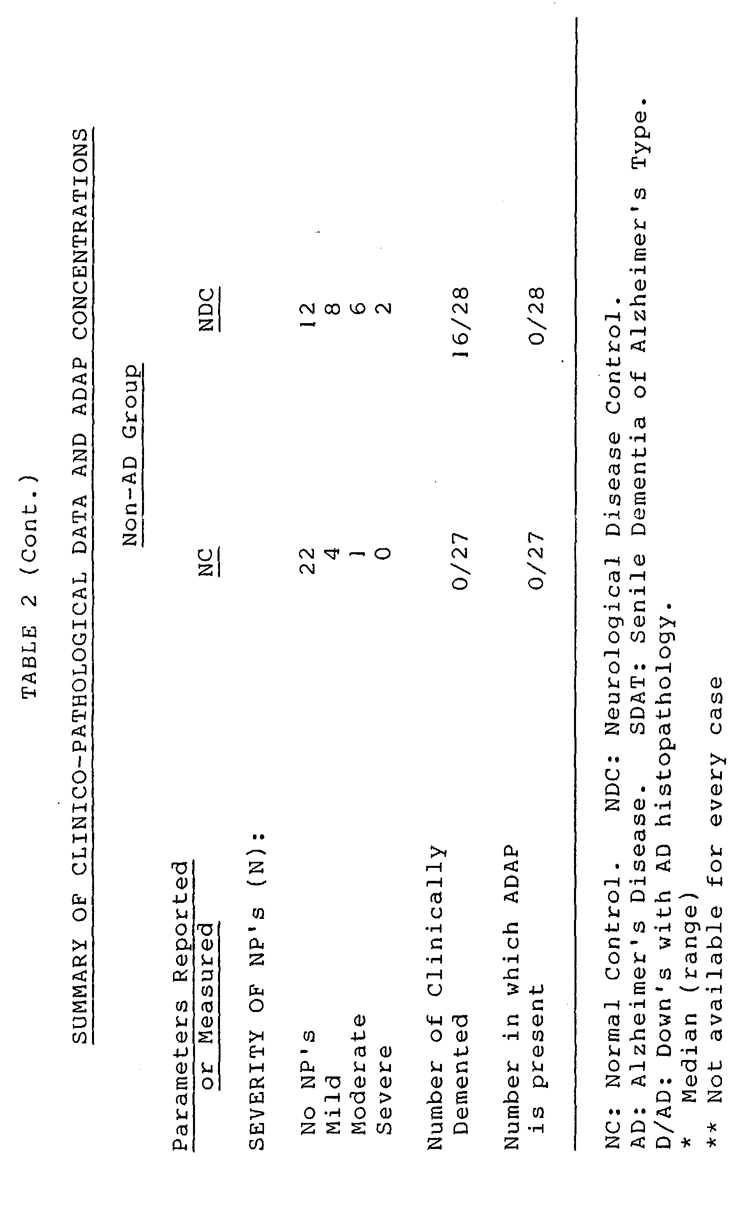

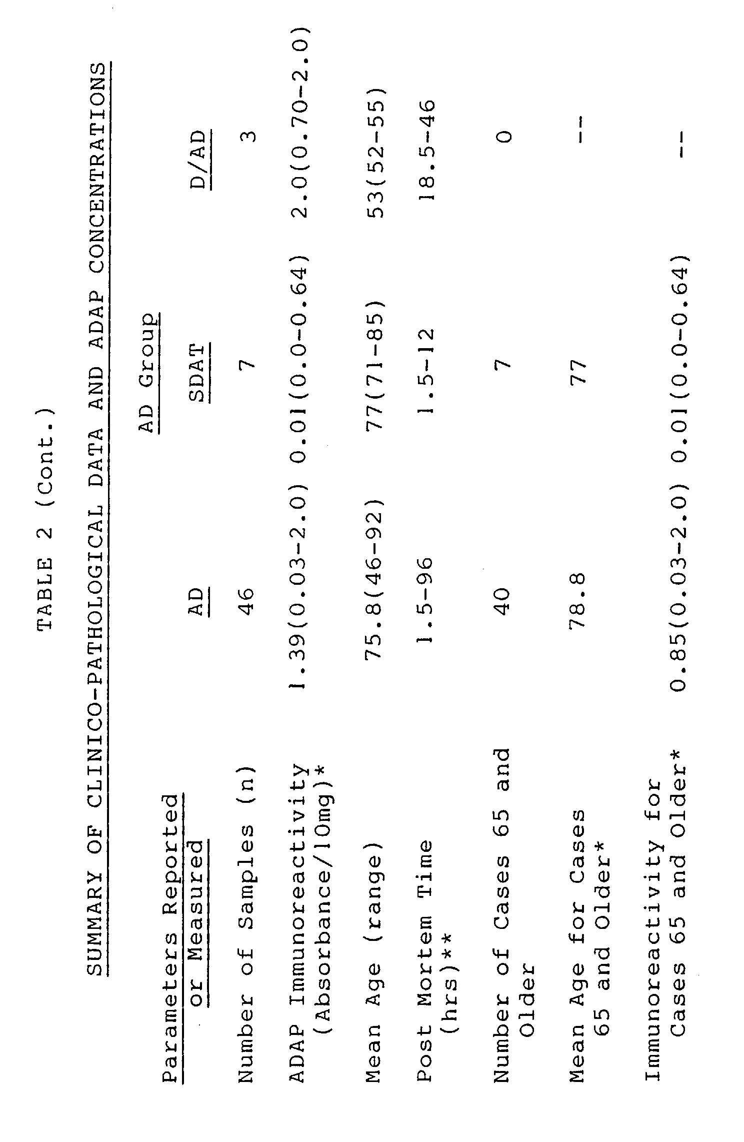

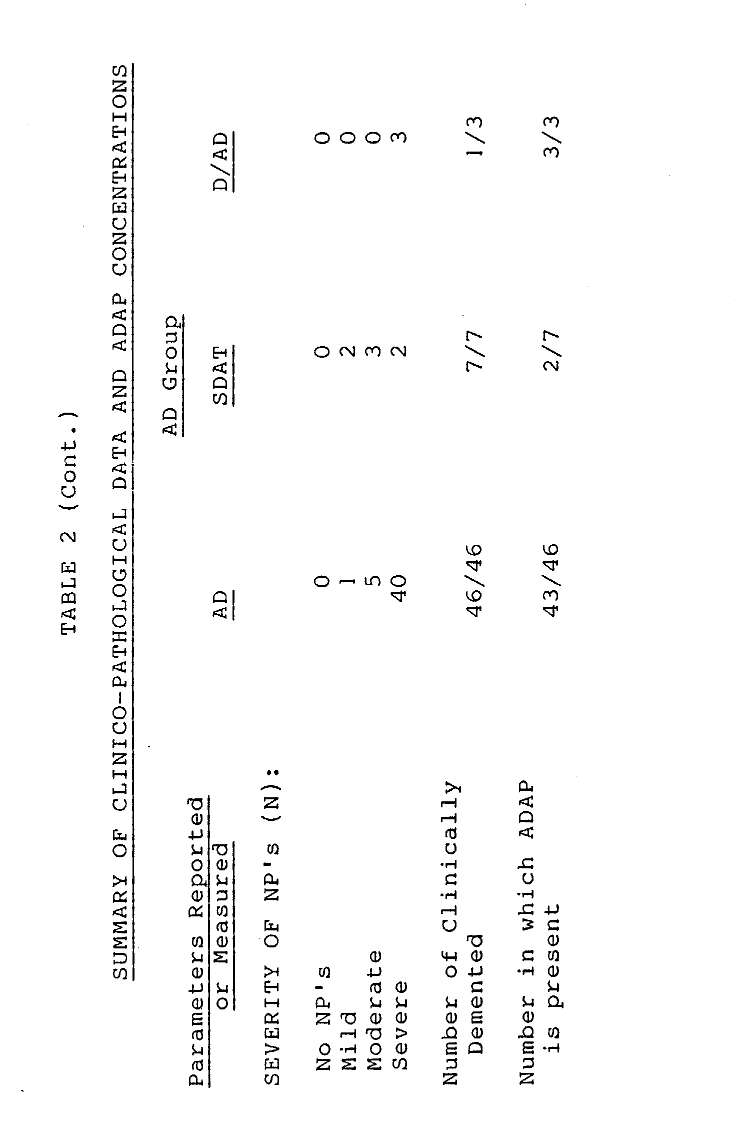

The Alzheimer's disease group had significantly greater ADAP concentrations (expressed as per protein or per 10 mg of tissue weight) than both the normal and other neurological disease groups. In fact, the normal brain group and the group of other neurological diseases essentially had no detectable ADAP. The ADAP concentrations in AD category had a median of 1.39 and ranged from 0.03 to 2.0 absorbance/10 mg tissue. The D/AD category mimicked AD, but the SDAT category demonstrated a wide range of the ADAP concentrations (from 0 to 0.64). The ADAP levels in NC and NDC categories were not significantly different (Student test-t, p |0.797). In contrast, ADAP level in the AD Group (n=56) was higher than non-AD group (n=55) using Student-t test (p 0.0005). When the ADAP concentrations in various categories were recalculated using cases 65 and older, the maximum values were actually reduced. The AD group had an overall median of 0.77 and ranged 0.0 to 2.0 absorbance units per 10 mg. tissue.

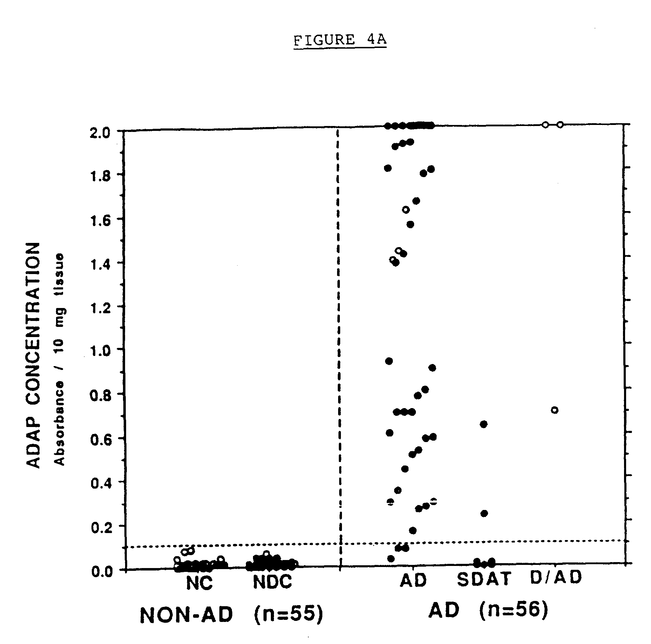

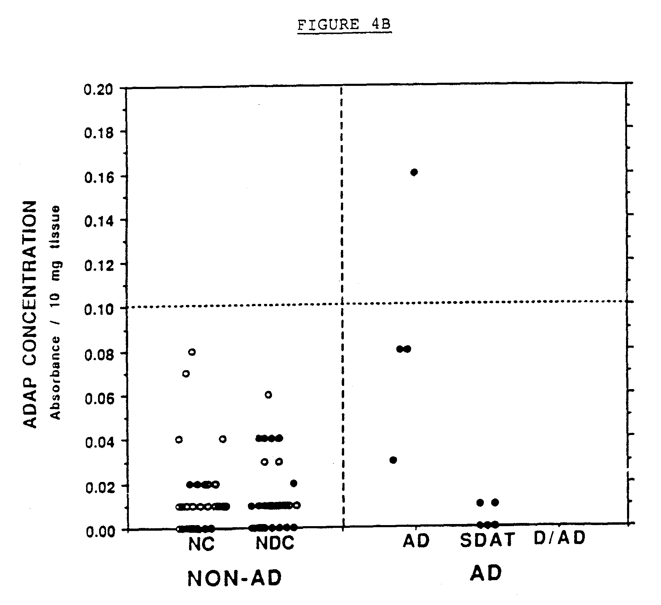

Figure 4 exhibits a scattergram plot of the ADAP concentration for the AD/SDAT versus the combined normal and other neurological group at two different scales. The group of normal brains and the group of other neurological diseases are presented together in Figure 4 because of the lack of difference in the ADAP concentrations (Dunnet's Test, p 0.05). An absorbance value of 0.1 was used as an empirically derived cut off between Normal range and AD range. ADAP concentrations for all non-AD cases fell under this line whereas ADAP concentrations for 48 out of 56 cases in AD group fell above this line. Only three cases in AD category overlapped with NC and NDC cases. It is noteworthy that there was no increase in ADAP levels in NDC category even though dementia was observed in 16 of a total of 28 cases. D/AD category also had ADAP levels comparable to AD, but SDAT cases had ADAP concentrations in both Normal range and AD range.

The data also was analyzed by a stepwise multiple regression analysis with forward selection technique (using dummy variables for diagnosis, gender, and pathological findings) and by Dunnet's Test when comparing the AD/SDAT and the other neurological diseases groups' ADAP levels to those of the normal group. The regression analysis considered the variables for diagnosis, age, sex, post-mortem delay and pathological report and explained the variance of the ADAP concentration (separate statistical analysis for each ADAP concentration unit category). The two dependent variables were ADAP concentration expressed as absorbance/mg protein and as absorbance/10 mg wet tissue. Fifteen percent (15%) of the variation of the former and 39% of the latter was explained (p=0.0001) by the diagnosis category alone. The variation was further explained by considering the neurofibrillary tangles and neuritic plaque reports to the extent of 7 and 5% (p 0.01), respectively. Finally, age was found to contribute 4 and 3%, respectively, to each variation but was inversely correlated (p 0.05). Thus, the total correlation coefficient (R2) was found to be 26 and 46%, respectively. This inverse correlation due to age was surprising, but may be explained by duration of disease (for which the data was not available).

There were several striking inconsistencies in the relationship (i.e., correlation of diagnosis to ADAP concentrations) which might be explained by a reported lack of correlation between neuritic plaque and neurofibrillary tangle counts to Alzheimer's disease clinical findings. (Hence a possible misdiagnosis in either direction or perhaps on the basis of duration of disease; the duration of the disease probably was not accurately determined for most of the patients since there is no systematic way in which onset of the disorder is reported.)

More specifically, the results indicate that the biochemical assay of ADAP (ALZ-EIA) produced no "false positives" in either NC or NDC cases. There were 8 "false negatives" in 56 cases in AD Group (specificity 85. 7%), but only 3 in 46 cases in AD category. There were 5 "false negatives" in SDAT category and none in D/AD category.

Upon further examination of the pathology reports, it was observed that the case with 0.03 absorbance/10 mg tissue (lowest AD) was 83 years old, had moderate NP, was initially labeled as SDAT, and more importantly had a brain tumor. One of the other two "false" negatives had a reported post-mortem time of 96 hours (highest in the study) and post-mortem time for the third one was not reported (not available), but it also was initially designated as SDAT. The SDAT cases, by convention, would have been diagnosed as AD if the patients had been under 65 years old at the onset of the disease. The histopathological reports of the 5 "false" negatives in the SDAT group were as follows: (P1) mild NP; (P2) moderate NP, cerebellar tumor; (P3) mild NP, Lewy bodies; (P4) moderate NP, no NFT, Lewy bodies; (P5) and severe NP. However, all Down's cases had Alzheimer's like histopathology (severe NP's and NFT's) and were positive for ADAP.

Moreover, while Alzheimer's Disease is associated with dementia, neuritic plaques and aging, dementia alone was not associated with an increase in ADAP concentration, because there were 24 demented cases (16 NDC's, 3 AD's, 5 SDAT's) in the normal range for ADAP (Table 2 and Figures 4A/B). Furthermore, NP's were not always associated with the presence of ADAP; there were 29 cases (5NC's, 16NDC's, 3AD's, 5 SDAT's) with NP's which had practically no detectable ADAP levels (Table 2, and Figure 4A/B).

To examine the concentration of ADAP as a function of age, the data pertaining to ADAP concentrations were recalcualted for the 65 and older cases. Based on this anaylsis, the maximum value of ADAP in 65 and older cases was actually lower in NC and NDC categories, and the median in AD category was decreased from 1.39 to only 0.85 for 65 and older cases (Table 2). Furthermore, in Figures 4A/B, the ADAP concentrations for 65 and older cases are presented in filled circles and for under 65 cases in open circles; there is no age related pattern apparent in the Figures. In fact, the highest values of ADAP immunreactivity in both NC and NDC categories correspond to cases under 65 (open circles, Figures 4A/B). The Spearman Correlation Test showed a 15% (no significant) probability of ADAP concentration being negatively correlated with age. The oldest NDC case was a 90 year old female with multi-infarct dementia showing moderate NP's, and yet with ADAP concentration of 0.01 absorbance unit/10 mg tissue, while the youngest AD case was 46 years old and had an ADAP concentration of more than 2.0.

It is apparent from the data that the claimed immunoassay distinguishes AD/SDAT from both normal and other neurological diseases. Furthermore, based on this data, clinical dementia, NP's, and old age per se do not appear to be associated with the increased ADAP levels in the AD group. Thus, using this assay, regional variations can be examined, as well as the relationship of the ADAP concentration to duration of the disease.

## Claims

1. A hybridoma cell line identified as ATCC No.HB9205.

2. A monoclonal antibody produced by a hybridoma cell line of Claim 1.

3. A protein preparation comprising an antigen immunologically reactive with the antibody of Claim 2, the preparation being obtainable by the process comprising:
(a) homogenizing a sample of cortical brain tissue in a Tris homogenization buffer; and
(b) removing particulate matter from the homogenate of step (a).

4. A method for determining Alzheimer's disease in an individual comprising:
(a) contacting a sample obtained from the individual with a monoclonal antibody produced by a hydridoma cell line identified as ATCC No. HB 9205 such that a first antibody-antigen complex is formed; and
(b) measuring the amount of the complex.

5. A method of Claim 4, wherein the sample is selected from cerebrospinal fluid, brain tissue extract, and blood.

6. A method of either Claim 4 or Claim 5, wherein the antibody is attached to a solid matrix.

7. A method according to any of Claims 4 to 6, further comprising the step of contacting the first complex with a second antibody immunologically reactive with a second antigenic determinant found in the brain tissue of individuals having Alzheimer's disease such that a second antibody-antigen complex is formed.

8. A method according to Claim 7, wherein the second antigenic determinant may be the same or different than the first antigenic determinant.

## Patentansprüche

1. Eine Hybridomazelllinie identifiziert als ATCC Nr. HB9205.

2. Ein monoklonaler Antikörper hergestellt durch die Hybridomazelllinie des Anspruchs 1.

3. Eine Proteinpräparation umfassend ein Antigen immunologisch reaktiv mit dem Antikörper von Anspruch 2, wobei die Präparation erhältlich ist durch das Verfahren umfassend:
(a) Homogenisieren einer Probe von kortikalem Hirngewebe in einem Tris-Homogenisierungspuffer und
(b) Entfernen bestimmter Stoffe aus dem Homogenat von Schritt (a).

4. Ein Verfahren zum Nachweis der Alzheimer-Krankheit in einem Individuum umfassend:
(a) Inkontaktbringen einer Probe, erhalten von dem Individuum mit einem monoklonalen Antikörper, hergestellt durch eine Hybridomazelllinie identifiziert als ATCC Nr. HB9205 so, daß ein erster Antikörper-Antigen-Komplex gebildet wird und
(b) Messen der Menge des Komplexes.

5. Ein Verfahren nach Anspruch 4, worin die Probe ausgewählt ist aus Cerebrospinalflüssigkeit, Hirngewebeextrakt und Blut.

6. Ein Verfahren nach Anspruch 4 oder 5, worin der Antikörper an eine feste Matrix gebunden ist.

7. Ein Verfahren nach einem der Ansprüche 4 bis 6, weiter umfassend den Schritt des Inkontaktbringens des ersten Komplexes mit einem zweiten Antikörper imunologisch reaktiv mit einer zweiten antigenen Determinante, die in dem Hirngewebe von Individuen mit Alzheimer-Krankheit gefunden wird, so daß ein zweiter Antikörper-Antigen-Komplex gebildet wird.

8. Ein Verfahren nach Anspruch 7, worin die zweite antigene Determinante gleich oder verschieden wie die erste antigene Determinante sein kann.

## Revendications

1. Lignée cellulaire d'hybridomes identifiée comme étant ATCC No. HB9205.

2. Anticorps monoclonal produit par une lignée cellulaire d'hybridomes selon la revendication 1.

3. Préparation protéinique comprenant un antigène immunologiquement réactif avec l'anticorps selon la revendication 2, la préparation pouvant être obtenue par le procédé comprenant:
(a) l'homogénéisation d'un échantillon de tissu cérébral cortical dans un tampon d'homogénéisation Tris; et
(b) l'élimination de la matière en particules du produit d'homogénéisation de l'étape (a).

4. Procédé pour la détermination de la maladie d'Alzheimer chez un individu, comprenant:
(a) la mise en contact d'un échantillon obtenu à partir de l'individu avec un anticorps monoclonal produit par une lignée cellulaire d'hybridomes identifiée comme étant ATCC No. HB 9205, de façon à former un premier complexe anticorps-antigène; et
(b) la mesure de la quantité du complexe.

5. Procédé selon la revendication 4, dans lequel l'échantillon est choisi parmi du liquide céphalorachidien, de l'extrait de tissu cérébral et du sang.

6. Procédé selon la revendication 4 ou la revendication 5, dans lequel l'anticorps est fixé à une matrice solide.

7. Procédé selon l'une quelconque des revendications 4 à 6, comprenant en outre l'étape de mise en contact du premier complexe avec un second anticorps immunologiquement réactif avec un second déterminant antigénique trouvé dans le tissu cérébral des individus ayant la maladie d'Alzheimer de façon à former un second complexe anticorps-antigène.

8. Procédé selon la revendication 7, dans lequel le second déterminant antigénique peut être identique au premier déterminant antigénique ou différent de celui-ci.
